# EUROPEAN PATENT SPECIFICATION

(11) **EP 0 673 392 B1**
(45) Date of publication and mention of the grant of the patent: **02.04.2003**
(21) Application number: 93921202.3
(22) Date of filing: 27.08.1993
(51) Int. Cl.: G01N 33/542, G01N 33/72

(54) **METHOD OF PREFERENTIAL LABELLING OF PHYCOBILIPROTEIN WITH AMINE-REACTIVE DYE FOR USE IN A MULTIPLE COLOR ASSAY**
VERFAHREN ZUR BEVORZUGTEN MARKIERUNG VON PHYCOBILIPROTEIN MITTELS AMINREAKTIVEM FARBSTOFF ZUR VERWENDUNG IN EINEM MEHRFARBENTEST
PROCEDE DE MARQUAGE PREFERENTIEL DE LA PHYCOBILIPROTEINE A L'AIDE D'UN COLORANT AMINOREACTIF DESTINE A ETRE UTILISE DANS UN TITRAGE MULTICOLORE

(30) Priority: 03.09.1992 US 940026; 11.12.1992 US 990861
(43) Date of publication of application: 27.09.1995
(73) Proprietor: Coulter International Corporation, Miami, Florida 33196 (US)
(72) Inventor: GUPTA, Ravinder K., Pembrook Pines, FL 33024 (US)
(74) Representative: Perry, Robert Edward
(86) International application number: US9308046
(87) International publication number: WO94005701

(56) References cited:
- WO-A-93/03062
- US-A- 4 542 104
- US-A- 4 666 862
- Becton-Dickinson Technical Brochure, Catalog No. 9026, issued 1990, "Steptavidin DuoCHROME", see the entire brochure.
- BioChromatography, Volume 1, issued 1986, K.M. GOODING, "High-Performance Liquid Chromatography of Proteins-A Current Look at the State of the Technique", pages 34-40, see page 37.
- Biophys. J., Volume 43, issued September 1983, A.N. GLAZER et al., "Fluorescent Tandem Phycobiliprotein Conjugates", pages 383-386, see page 384.
- Nucleic Acids Research, Volume 13, no. 7, issued 1985, L.M. SMITH, "The Synthesis of Oligonucleotides Containing an Aliphatic Amino Group at the 5' Terminus: Synthesis of Fluorescent DNA Primers for Use in DNA Sequence Analysis", pages 2399-2412, see pages 2402-2405.
- Cytometry, Volume 11, issued 1990, P.L. SOUTHWICK et al., "Cyanine Dye Labeling Reagents-Carboxymethylindocyanine Succinimidyl Esters", pages 418-430, see pages 418-419.
- Bioconjugate Chemistry, Volume 4, No. 2, issued March/April 1992, R.B. MUJUMDAR et al., "Cyanine Dye Labeling Reagents: Sulfoindocyanine Succinimidyl Esters", pages 105-111, see page 109.

## Description

### TECHNICAL FIELD

This invention relates generally to electron donor-acceptor conjugates suitable for use in multiple color assay methods, particularly to a method of producing phycobiliprotein-dye conjugates suitable for such use.

### BACKGROUND ART

The technique of fluorescence was first introduced by Coons in 1941. He used a blue fluorescing anthracene compound coupled to pneumococcus antiserum to detect bacterial antigens in tissue section. Subsequent to this initial discovery, many fluorescing materials have been investigated, but only two, the fluorochromes fluorescein and rhodamine, are widely used, particularly in the form of fluorescein isothiocyanate (FITC) and tetramethylrhodamine isothiocyanate (TRITC) respectively. FITC covalently binds to proteins at alkaline pH through the epsilon (ε) amino residues of lysine and through terminal amino groups. FITC's adsorption maximum is at 490-495 nm and it emits its characteristic green color at 517nm. TRITC likewise binds to proteins, has its absorption maximum at 541 nm and emits its characteristic red color at 572 nm.

Fluorescence is the emission of light of one wavelength (color) by a substance that is being irradiated by light of a different wavelength. The emitted light is always of lower energy, hence longer wavelength, then the incident light. In clinical use, the strength of the fluorescence is dependent on the efficiency with which the fluorochrome transforms incident light into emitted light, the amount of dye present in the specimen under observation and the intensity of the incident light. The dye known as Texas Red (sulforhodamine 101 sulfonyl chloride or sulforhodamine acid chloride) has previously been investigated for clinical use in conjugation with phycoerythrins, but major problems were encountered. These problems were low fluorescent efficiency, inadequate energy transfer from the phycoerythrin to Texas Red and the instability of the phycoerythrin-Texas Red conjugate. Phycoerythrin-Texas Red conjugates are desirable, however, because the overlap of their absorption and emission spectra have the potential to give a strong fluorescence signal.

Low fluorescent efficiency occurs whenever fluorescent chromophores are spatially adjacent to each other. It is usually called concentration quenching. See R. P. Hughland, "Excited States of Biopolymers", R. F. Steins, Ed., p 47 (Plenum Press, New York, 1983). However, high levels of labelling, resulting in chromophores being spatially adjacent to each other, are required in order to assure adequate energy (electron) transfer from the phycoerythrin to the acceptor dye chromophore. The net result is that the trade off required by the opposing effects results in less than optimal performance. Recently, A. N. Glazer et al. have covalently linked a phycoerythrin to an allophycocyanin to produce a highly fluorescent tandem conjugate with an energy transfer efficiency of 90%. See A. N. Glazer et al., T.I.B.S, 9:423 (1984); Biophysics J., 43,386-386 (1983); and U.S. Patent No. 4,542,104 (See also U.S. Patent No. 4,520,110 to L. Stryer et al. describing the use of phycobiliproteins as fluorescent probes for the analysis and separation of molecules and cells). However, forming a conjugate from two naturally occurring pigments derived from algae is much different from conjugating a synthetic dye such as Texas Red. In fact, the procedures usually followed for conjugating reactive dyes to proteins does not work with phycoerythrin-Texas Red. Using such procedures, one obtains a complex with a low energy transfer efficiency at low levels of labelling or fluorescence quenching at high levels of labelling. Texas Red forms a conjugate with a phycoerythrin by reaction of its sulfonyl or acid chloride moiety with an amine group of phycoerythrin or other phycobiliprotein.

Phycobiliprotein/amine-reactive dye conjugates are known and some, for example, phycoerythrin-Texas Red conjugates, are commercially available. For example, the phycoerythrin-Texas Red conjugate known as DuoCHROME ™ is available (bound to streptavidin) from Becton Dickinson Immunology Systems, Mountain View, California (Catalog No. 9026). The available conjugates do not have a uniform phycoerythrin-Texas Red ratio throughout the individual conjugate members. Overlabelled and underlabelled species are present, as well as species having the desired or optimum degree or range of labelling. Consequently, energy transfer/quenching problems can arise, depending upon the distribution of labelled species within the entire sample.

WO-A-93/03062 is in the state of the art according to Article 54(3) EPC; it discloses preparing a phycobiliprotein-dye conjugate in the presence of a salt which causes a hydrophobic intramolecular re-arrangement of the phycobiliprotein, thereby exposing more hydrophobic sites for binding to the dye. The given phycobiliprotein is phycoerythrin and the given dye is Texas Red. The conjugate is useful in multiple colour fluorescence assays, without requiring the use of multiple exciting sources.

### SUMMARY OF THE INVENTION

According to the present invention, a method for producing a phycobiliprotein/amine-reactive dye conjugate, wherein the phycobiliprotein is labelled with a dye selected from sulfonic and carboxylic acids and their acid chlorides and esters, carboxyfluorescein succinimidyl esters, fluorescein-5-isothiocyanate and fluorescein-6-isothiocyanate, provided that the phycobiliprotein is not phycoerythrin when the dye is Texas Red, comprises reacting the dye with a phycobiliprotein in the presence of a salt, removing excess dye, and separating the conjugate having a desired degree of labelling, from overlabelled and underlabelled conjugates, by chromatographic means.

This invention solves the energy transfer/quenching problem encountered in the preparation of phycobiliprotein/amine-reactive dye (PARD) conjugates, by preferentially labelling sites dose to the chromophore regions of the phycobiliprotein with the dye, and separating overlabelled and underlabelled conjugates from conjugates having the desired degree of labelling by chromatographic methods; for example, by exploiting the differences in hydrophobic character of conjugates having different degrees of labelling.

In the invention, the dye is reacted with the phycobiliprotein, such as phycoerythrin or an allophycocyanin, in the presence of the salt, to expose a multiplicity of sites in its hydrophobic region with which said dye can bind to form the desired conjugate. The reaction is controlled as to the anion of the selected salt, permitted time of reaction and temperature. Conjugates having the preferred degree of phycobiliprotein/amine-reactive dye conjugation are separated from overlabelled and underlabelled conjugates by hydrophobic interaction chromatography. Alternatively, hydrophobic interaction chromatography may be used to separate conjugates having the desired degree of labelling from a reaction mixture which did not use the selective salts taught herein.

### DESCRIPTION OF THE INVENTION

The first feature of this invention, preferential site labelling, makes it possible to obtain a satisfactory level of energy transfer from a phycobiliprotein to an amine-reactive dye even at low levels of dye conjugation by bringing the dye and the chromophore of the phycobiliprotein into close proximity. This is accomplished by making use of the hydrophobic tetrapyrrole (bilin) chromophores that biliproteins are known to possess. See R. McColl and D. Guard-Frier, Phycobiliproteins. Chapter 1, C.R.C. Press (1987). Specifically, when certain anions commonly used in some "salting-out" processes are added to a phycobiliprotein containing buffer solution, they cause the phycobiliprotein to undergo an intramolecular structural rearrangement which "opens-up" or "exposes" hydrophobic sites on the protein by reducing steric hindrance about the site. As a result of this hydrophobic intramolecular rearrangement, the sites close to chromophores can more readily react with a reactive dye, such as Texas Red, to form a conjugate. The common ions used in this process may be any of the common ions used in "salting-out" processes, such as phosphate, acetate, citrate, sulfate, tartrate and the like. The preferred anions are sulfate, phosphate and acetate. The most preferred anion is sulfate because it has little or no effect on the pH of the solution. The exact amount of anion required in a given reaction is dependent on the particular phycobiliprotein undergoing reaction. For example, allophycocyanin requires the use of about 8% to 12% sodium sulfate. Using the principles taught herein, the optimal concentration of the selected salt can easily be determined. Overall, the optimal concentrations will range from 1% to 20%, e.g. 1 to 12%.

The phycobiliprotein and the amine-reactive dye are reacted together at a pH greater than 7 for a time in the range of 10 minutes and at a temperature of about 4°C to about 25°C prior to sampling to determine if an overall adequate phycobiliprotein-dye conjugation ratio has been reached. The preferred pH is greater than 8 and less than 12. The determination is carried out by chromatographically removing excess dye from a sample of the reaction mixture and spectroscopically determining an absorbance ratio, Aₓ/A_{y}, defined as the ratio of the intensity of the maximum absorption of the phycobiliprotein divided by the intensity of the maximum adsorption of the amine-reactive dye. The Aₓ/A_{y} value will differ for different PARD conjugates. The removal of the excess dye simultaneously removes excess salts such as the sodium sulfate preferably used to expose a phycobiliprotein's hidden hydrophobic sites.

An excess of amine-reactive dye is used in the claimed method. The initial molar ratio of amine-reactive dye to phycobiliprotein in the reaction mixture is in the range of about 5:1 to about 30:1.

A phycobiliprotein/amine-reactive dye conjugate is formed by reaction of an amino group on the phycobiliprotein with a reactive group present on the amine-reactive dye. The reactivity of phycobiliprotein amino groups is well known. For example, small biomolecules such as biotin have been attached to phycobiliproteins by reaction with an appropriate activated ester or sulfonyl chloride derivative. The reaction between phycobiliproteins and amine-reactive dyes is analogous to the biotin reaction and to the reactions of fluorescein isothiocyanate with the ε-amino residues of lysine and terminal amino groups previously mentioned. The preferred reactive moieties present on the amine-reactive dye are selected from the group consisting of sulfonic and carboxylic acids and their acid chlorides and esters. Specific examples of such dyes include 5- or 6-carboxyl-x-rhodamine succinimidyl esters, sulforhodamine 101 sulfonyl chloride, Lissemine rhodamine B sulfonyl chloride. Compounds such as fluorescein-5-isothiocyanate and fluorescein-6-isothiocyanate, are further examples of amine-reactive dyes.

Phycobiliproteins in general may be used according to the invention. In addition to phycoerythrin and allophycocyanin used in the Examples herein, C-phycocyanin, R-phycocyanin and phycoerythrocyanin, among others, may be reacted as described herein.

The separation of over-labelled and under-labelled PARD conjugate species from those having the desired degree of labelling was accomplished using hydrophobic interaction chromatography with an appropriate column medium like butyl TOYOPEARL™ (Toso Haas, Philadelphia, Pennsylvania). The PARD conjugate produced by this method can be used in conjunction with an antibody to stain different types of cell. The cells so stained will be dependent upon the choice of antibody. The importance of the invention lies in the fact that PARD conjugates provide for an additional color in fluorescence analysis with the use of only a single excitation wavelength, which wavelength is determined by the choice of the amine-reactive dye. For example, FITC, R-phycoerythrin and APC-FSE can be excited with a single excitation wavelength (laser) of 488nm to emit maximally at about 525,575, and 660 nm respectively. As a result of this feature, the expense of multiple excitation sources is eliminated.

The following Examples 1 and 3 illustrate the invention. Example 2 is comparative.

### Example 1 Reaction of Allophycocyanin (APC) with a Carboxyfluorescein Succinimidyl Ester (FSE).

6.25 ml (292.4 mg) APC solution (46.748 mg/ml in PBS, 2 mM EDTA) was treated by dropwise addition, with stirring, of a solution resulting from mixing PBS, 2 mM EDTA (6.17 ml), 1M potassium borate of pH 9.80 (7.3 ml) and 20 wt% sodium sulfate of pH 7.0 (8.76 ml). A 14-fold excess of FSE (25 mg/ml in anhydrous dimethylforamide) was added to the APC solution at room temperature (about 22°C). After 30 minutes, a 50 µL sample of the reaction mixture was withdrawn, excess dye removed on a SEPHADEX® G-50 column in PBS which was 2 mM in EDTA, and the protein A₄₉₆/A₆₅₂ value for the protein peak was checked.

If the A₄₉₆/A₆₅₂ ratio is less than 0.5 after 60 minutes or more reaction time, an additional aliquot of FSE was added to the reaction mixture. When an A₄₉₆/A₆₅₂ value in the range of 0.5-0.7 is achieved, the reaction is stopped by removing excess dye on a SEPHADEX® G-50 column in PBS, 2 mM EDTA. Alternatively, a quenching agent is added to the reaction mixture prior to passage through the SEPHADEX® column. The protein peak is collected and the APC-FSE conjugate mixture is further fractionated using hydrophobic interaction chromatography on a butyl 650S (Toso Haas) column using a reverse gradient (4% to 0%) sodium sulfate in 100 mM potassium phosphate, 2 mM EDTA of pH 7.0 ± 0.1, followed by steps of 50 mM potassium phosphate, 2 mM EDTA of pH 7.0 ± 0.1, and lastly, PBS, 2 mM EDTA. Fractions showing high energy transfer efficiency and high fluorescence were found to have an A₄₉₆/A₆₅₂ ratio of about 0.4-0.6. These were pooled, dialyzed against PBS, 2 mM EDTA and concentrated. The yield was 54%. The conjugate was used without further purification.

### Example 2 APC-FSE Conjugate Prepared Without Using Selected Salts.

APC and FSE are reacted as in Example 1, but without the addition of the selected salt. The reaction time is extended within the range of 0.5 to 5.0 hours after the addition of FSE to APC is completed. The reaction mixture is then passed through a SEPHADEX® G-50 column and is subsequently fractionated by hydrophobic interaction chromatography. Fractions having A₄₉₆/A₆₅₂ in the range of 0.4-0.6 are collected, dialyzed and concentrated, and may be used without further purification. The yield is lower than in Example 2 where selected salts were used and is estimated to be about 35% at a maximum.

### Example 3 Reaction of R-Phycoerythrin with the Amine-Reactive Dye Cy5.18-OSu.

1 ml (50 mg) of purified R-phycoerythrin solution (PE, 50 mg/ml 2mM EDTA), cooled to 0°C in an ice bath, was treated by dropwise addition, with stirring, with a 0° solution resulting from mixing a 1 M borate-PBS solution (pH 9.8, 3.125 ml), 2 mM EDTA (4.45 ml) and 20% Na₂SO₄ (pH 7.0, 0.625 ml). A 10-fold molar excess of Cy5.18-OSu, a sulfoindocyanine succinimidyl ester (hereafter CY, 1 mg/ml in PBS, 2 mM EDTA, available as Cy5™ from Biological Detection Systems, Inc., Rockville, Maryland; formula and structure described by R.B. Mujumdar et al., Bioconjugate Chemistry 4(2): 105-111 (1993)) was added to the resulting R-phycoerythrin solution. Reaction progress was monitored by periodically taking a 50 µL sample of the reaction mixture, desalting the sample on a SEPHADEX® G-50 column in PBS which is 2 mM in EDTA, collecting the protein peak and spectroscopically determining the protein A₅₆₅/A₆₆₅. If the value is more than 2.3 after 10 minutes of reaction, another 1- to 5-fold excess of CY, relative to the initial PE quantity, was added to the reaction mixture. About 1-4% of a selected salt is used in the preparation of PE/CY.

When A ₅₆₅/A₆₆₅ value is in the range of 1.6-2.3, the reaction mixture was desalted on a SEPHADEX® G-50 column in PBS which is 2 mM in EDTA. Alternatively a quenching agent such as hydroylamine or 2-aminoethanol is added to the reaction mixture before desalting on SEPHADEX® G-50 as described. The protein peak is collected and the PE-CY conjugate is further fractionated using hydrophobic interaction chromatography on a Butyl 650S column using a reverse gradient (4.5% to 0% w/v) sodium sulfate solution which is 100 mM in potassium phosphate, 2 mM EDTA and pH 7.0 ± 0.1. The chromotographic fractions were checked for emissions at 575 nm (PE) and 665 nm (CY) using a 488 nm excitation source. the fractions having high energy transfer efficiency (E₅₇₅/Em₆₆₅ ≤ 0.15) and high energy transfer efficiency were combined, concentrated, dialysed against PBS, 2 mM EDTA and reconcentrated.

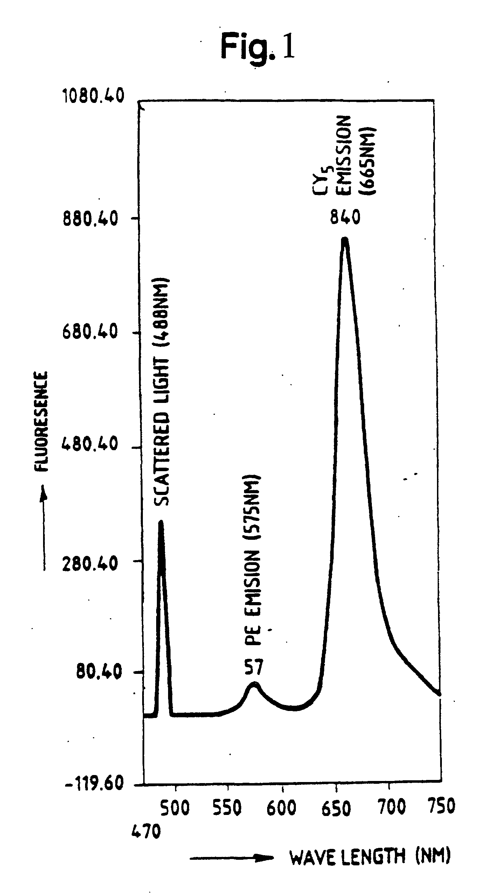

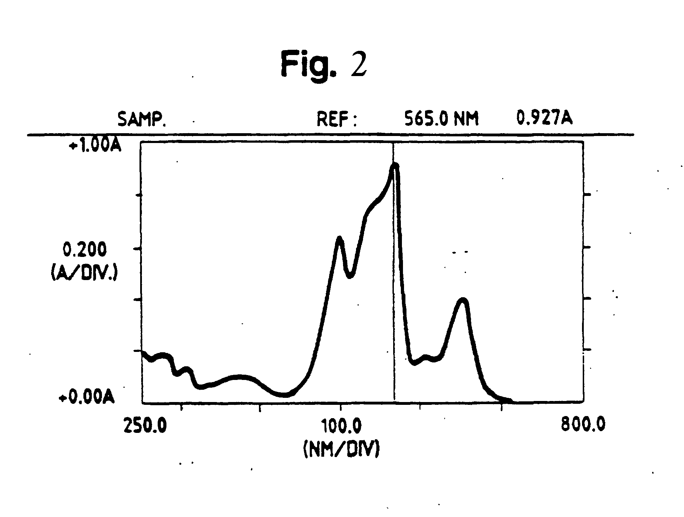

The emission spectrum of the R-phycoerythrin/Cy5.18-OSu conjugate, after excitation at 488 nm, is shown in Fig. 1 of the accompanying drawings, and shows about 93-96% energy transfer efficiency (Em₅₇₅/Em₆₆₅ = about 0.04 to about 0.07). The absorption spectrum is shown in Fig. 2.

The PE/CY product can then be conjugated to proteins such as antibodies by method known to those skilled in the art and used in various analytical methods such as flow cytometry.

## Claims

1. A method for producing a phycobiliprotein/amine-reactive dye conjugate wherein the phycobiliprotein is labelled with a dye selected from those having sulfonic or carboxylic acid moieties, or their acid chlorides or esters, carboxyfluorescein succinimidyl esters, fluorescein-5-isothiocyanate and fluorescein-6-isothiocyanate, provided that the phycobiliprotein is not phycoerythrin when the dye is Texas Red, the method comprising reacting the dye with a phycobiliprotein in the presence of a salt which effects the intramolecular rearrangement of said phycobiliprotein to expose reactive amino groups in hydrophobic sites on said phycobiliprotein which reacts, removing excess dye, and separating the conjugate having a desired degree of labelling, from overlabelled and underlabelled conjugates, by chromatographic means.

2. The method of claim 1, wherein the molar ratio of dye to phycobiliprotein at the start of the reaction is in the range of 5:1 to 30:1.

3. The method of claim 1 or claim 2, wherein the salt is one whose anion is selected from phosphate, sulphate, acetate, citrate and tartrate.

4. The method of any preceding claim, wherein the chromatographic means is hydrophobic interaction chromatography.

5. The method of any preceding claim, wherein the salt has a concentration in the range of 1% to 20%.

6. The method of claim 5, wherein the salt has a concentration in the range of 1% to 12%.

7. The method of any preceding claim, wherein the reaction is conducted at a pH greater than 7.

8. The method of claim 5, which comprises reacting a phycobiliprotein-containing solution, having a pH greater than 7, with a molar excess of the dye, at a temperature in the range of 4 to 25°C.

9. The method of any preceding claim, wherein the phycobiliprotein is phycoerythrin.

10. The method of any of claims 1 to 8, wherein the phycobiliprotein is allophycocyanin, C-phycocyanin, R-phycocyanin or phycoerythrocyanine.

11. The method of any preceding claim, wherein the dye is 5- or 6-carbonyl-x-rhodamine succinimidyl ester, sulforhodamine 101 sulfonyl chloride or L-issimine rhodamine β-sulfonyl chloride.

12. The method of claim 11, wherein the dye is Cy5.18-OSu.

13. The method of claim 12, wherein the reaction comprises reacting an excess of Cy5.18-OSu with phycoerythrin at a pH greater than 8, e.g. a in a buffer solution.

## Patentansprüche

1. Verfahren zur Herstellung eines Phycobiliprotein/Amin-reaktiven Farbstoff-Konjugats, worin das Phycobiliprotein mit einem Farbstoff markiert ist, der ausgewählt ist aus jenen mit Sulfon- oder Carbonsäure-Gruppen oder deren Säurechloriden oder Estern, Carboxyfluoresceinsuccinimidylestern, Fluorescein-5-isothiocyanat und Fluorescein-6-isothiocyanat, mit der Maßgabe, dass das Phycobiliprotein nicht Phycoerythrin ist, wenn der Farbstoff Texas Red ist, wobei das Verfahren das Umsetzen des Farbstoffs mit einem Phycobiliprotein in Anwesenheit eines Salzes, welches die intramolekulare Umlagerung des Phycobiliproteins unter Freilegung reaktiver Aminogruppen an hydrophoben Stellen am reagierenden Phycobiliprotein bewirkt, das Entfemen von überschüssigem Farbstoff und das Trennen des Konjugats mit einem gewünschten Markierungsgrad von übermarkierten und untermarkierten Konjugaten durch chromatographische Mittel umfasst.

2. Verfahren nach Anspruch 1, worin das Molverhältnis von Farbstoff zu Phycobiliprotein zu Beginn der Reaktion im Bereich von 5:1 bis 30:1 liegt.

3. Verfahren nach Anspruch 1 oder Anspruch 2, worin das Salz eines ist, dessen Anion aus Phosphat, Sulfat, Acetat, Citrat und Tartrat ausgewählt wird.

4. Verfahren nach irgendeinem vorhergehenden Anspruch, worin das chromatographische Mittel hydrophobe Wechselwirkungschromatographie ist.

5. Verfahren nach irgendeinem vorhergehenden Anspruch, worin das Salz eine Konzentration im Bereich von 1% bis 20% aufweist.

6. Verfahren nach Anspruch 5, worin das Salz eine Konzentration im Bereich von 1% bis 12% aufweist.

7. Verfahren nach irgendeinem vorhergehenden Anspruch, worin die Reaktion bei einem pH-Wert von größer als 7 durchgeführt wird.

8. Verfahren nach Anspruch 5, welches das Umsetzen einer Phycobiliprotein enthaltenden Lösung mit einem pH-Wert von größer als 7 mit einem molaren Überschuss des Farbstoffs bei einer Temperatur im Bereich von 4 bis 25°C umfasst.

9. Verfahren nach irgendeinem vorhergehenden Anspruch, worin das Phycobiliprotein Phycoerythrin ist.

10. Verfahren nach irgendeinem der Ansprüche 1 bis 8, worin das Phycobiliprotein Allophycocyanin, C-Phycocyanin, R-Phycocyanin oder Phycoerythrocyanin ist.

11. Verfahren nach irgendeinem vorhergehenden Anspruch, worin der Farbstoff 5- oder 6-Carboxyl-x-rhodaminsuccinimidylester, Sulforhodamin 101-sulfonylchlorid oder Lisseminrhodamin-β-sulfonylchlorid ist.

12. Verfahren nach Anspruch 11, worin der Farbstoff Cy5.18-OSu ist.

13. Verfahren nach Anspruch 12, worin die Reaktion die Umsetzung eines Überschusses von Cy5.18-OSu mit Phycoerythrin bei einem pH von mehr als 8, z.B. in einer Pufferlösung, umfasst.

## Revendications

1. Méthode de production d'un conjugué phycobiliprotéine/colorant réactif d'amine, où la phycobiliprotéine est marquée avec un colorant sélectionné parmi ceux ayant des fractions d'acide sulfonique ou carboxylique ou leurs chlorures d'acides ou esters, succinimidyl esters de carboxyfluorescéine, fluorescéine-5-isothiocyanate et fluorescéine-6-isothiocyanate, à condition que la phycobiliprotéine ne soit pas la phycoérythrine quand le colorant est Rouge Texas, la méthode comprenant la réaction du colorant avec une phycobiliprotéine en présence d'un sel qui effectue le réarrangement intramoléculaire de ladite phycobiliprotéine pour exposer des groupes aminés réactifs dans des sites hydrophobes sur ladite phycobiliprotéine qui réagit, l'enlèvement du colorant en excès et la séparation du conjugué ayant un degré souhaité de marquage, des conjugués surmarqués et sous-marqués, par un moyen chromatographique.

2. Méthode de la revendication 1, où le rapport molaire du colorant à la phycobiliprotéine au début de la réaction est dans la gamme de 5:1 à 30:1.

3. Méthode de la revendication 1 ou de la revendication 2, où le sel en est un dont l'anion est sélectionné parmi phosphate, sulfate, acétate, citrate et tartrate.

4. Méthode de toute revendication précédente, où le moyen chromatographique est une chromatographie par interaction hydrophobe.

5. Méthode de toute revendication précédente, où le sel a une concentration dans la gamme de 1% à 20%.

6. Méthode de la revendication 5, où le sel a une concentration dans la gamme de 1% à 12%.

7. Méthode de toute revendication précédente, où la réaction est entreprise à un pH plus grand que 7.

8. Méthode de la revendication 5, qui comprend la réaction d'une solution contenant de la phycobiliprotéine ayant un pH plus grand que 7 avec un excès molaire du colorant, à une température dans la gamme de 4 à 25°C.

9. Méthode de toute revendication précédente, où la phycobiliprotéine est la phycoérythrine.

10. Méthode de l'une quelconque des revendications 1 à 8, où la phycobiliprotéine est l'allophycocyanine, la C-phycocyanine, la R-phycocyanine ou la phycoérythrocyanine.

11. Méthode de toute revendication précédente, où le colorant est le succinimidyl ester de 5- ou 6-carbonyl-x-rhodamine, le chlorure de sulfonyle sulforhodamine 101 ou le chlorure de β-sulfonyl L-issime rhodamine.

12. Méthode de la revendication 11, où le colorant est Cy5.18-OSu.

13. Méthode de la revendication 12, où la réaction comprend la réaction d'un excès de Cy5.18-OSu avec de la phycoérythrine à un pH plus grand que 8, e.g., dans une solution tampon.
